# NEUE EUROPÄISCHE PATENTSCHRIFT

(11) **EP 2 384 822 B2**
(45) Veröffentlichungstag und Bekanntmachung des Hinweises auf die Entscheidung über den Einspruch: **24.06.2020**
(45) Hinweis auf die Patenterteilung: 14.09.2016
(21) Anmeldenummer: 11003139.0
(22) Anmeldetag: 14.04.2011
(51) Int. Cl.: B05C 17/005, B05C 17/01, B65D 81/32

(54) **Kartuschenverschluss und Kartusche mit einem solchen Verschluss**
Cartridge lock and cartridge with such a lock
Fermeture de cartouche et cartouche dotée d'une telle fermeture

(30) Priorität: 04.05.2010 DE 102010019219
(43) Veröffentlichungstag der Anmeldung: 09.11.2011
(73) Patentinhaber: Heraeus Medical GmbH, 61273 Wehrheim (DE)
(72) Erfinder: Vogt, Sebastian, Dr., 99092 Erfurt (DE); Büchner, Hubert, Dr., 90491 Nürnberg (DE); Schnieber, Tim, 60439 Frankfurt (DE)
(74) Vertreter: Schultheiss & Sterzel Patentanwälte PartG mbB

(56) Entgegenhaltungen:
- EP-A1- 0 261 466
- WO-A1-2005/016783
- WO-A1-2006/005213
- WO-A1-2009/061884
- WO-A2-2007/104037
- DE-A1- 3 811 954
- DE-A1- 19 961 485
- DE-U1- 9 102 635
- DE-U1- 9 102 635
- DE-U1- 29 709 383
- DE-U1-202006 014 087
- DE-U1-202008 009 692
- FR-A- 650 157
- JP-A- 2009 291 234
- US-A- 4 846 373
- US-A- 5 027 981
- US-A- 5 380 295
- US-A- 5 566 860
- US-A- 5 893 486
- US-A- 5 944 226
- US-A1- 2005 150 916
- US-A1- 2007 051 750
- US-A1- 2007 166 660

## Beschreibung

Die Erfindung betrifft ein Kartuschensystem zum Applizieren eines Materials, insbesondere eines medizinischen Zements, umfassend zumindest eine Kartusche, wobei die zumindest eine Kartusche Kartuschenwände und jeweils einen Kartuschenkopf mit jeweils zumindest einer Öffnung im Kartuschenkopf umfasst oder umfassen und am Kartuschenkopf oder den Kartuschenköpfen eine Halterung angeordnet ist.

Reaktive pastöse Zwei- oder Mehrkomponentensyteme müssen nach ihrer Herstellung bis zur Applikation getrennt aufbewahrt werden, um vorzeitige, unbeabsichtigte Reaktionen der Komponenten zu verhindern. Kartuschensysteme für die Applikation von pastösen Zwei- oder Mehrkomponentensystemen sind seit Jahrzehnten bekannt. Beispielhaft seien dafür die Dokumente CH 669 164 A5, EP 0 607 102 A1, EP 0 236 129 A2, DE 3 440 893 A1, US 4,690,306 A, US 2009/062808 A1, EP 0 787 535 A1, WO 2006/005 206 A1, EP0 693 437 A1, EP 0 294 672 A, EP 0 261 466 A1 und EP 2 008 707 A1 genannt. Nach der Befüllung der Kartuschen mit reaktiven Pasten müssen diese sicher bis zur Applikation verschlossen bleiben. Die Vermischung der pastösen Zwei- oder Mehrkomponentensysteme erfolgt unmittelbar bei der Applikation üblicherweise mit Hilfe von statischen Mischern. Exemplarisch seien dafür die Dokumente GB 1,188,516 A, US 2,125,245 A, US 5,968,018 A, US 4,068,830 A, US 2003/179648 A1, EP 0 664 153 A1 und EP 0 289 882 A1 angeführt. Bewegliche Kolben dichten dabei die Kartuschenböden ab und werden anschießend bei der Applikation zum Auspressen der Pasten verwendet. Ein Kartuschensystem mit einer Rastvorrichtung an einem Förderkolben einer Kartusche ist aus der EP 2 008 707 A1 bekannt.

Zum Verschließen eines Kartuschenkopfs werden eine Reihe von Lösungen vorgeschlagen. Ein einfaches, aber sehr wirksames Prinzip besteht darin, den Kartuschenkopf mit einem drehbaren Verschluss zu verschließen (EP 0 431 347 A1, DE 2 017 292 A1, US 3,215,298 A). Der Verschluss wird vor der Applikation herausgedreht. Anschließend wird ein Austragsrohr in ein Gewinde am Kartuschenkopf eingedreht oder mit einem, ein Gewinde nachbildendem Zapfensystem fixiert. Nachteilig ist hieran, dass der Anwender zweimal Drehbewegungen ausführen muss, bis das Pastenmaterial ausgetrieben werden kann. Weiterhin kann es passieren, dass der Verschluss herausgedreht wird und das Austragsrohr erst später aufgesetzt wird. Zwischen dem Öffnen der Kartuschen und dem Einsetzen des Austragsrohrs kann es, insbesondere wenn flüchtige Substanzen in den Pasten enthalten sind, zur Verdunstung von Bestandteilen der Pasten kommen.

Der gegenwärtig sehr häufig, besonders in der Klebstoff- und Dichtungsmittelindustrie, verwendete Verschluss basiert darauf, dass am Kartuschenkopf das Wandmaterial der Kartusche sehr dünn ausgebildet ist, so dass diese Wandung leicht durchstochen werden kann. Nachteilig ist daran, dass beim Durchstoßen Partikel der Wandung abgelöst werden können, die dadurch in das pastöse Material gelangen.

In der Medizin werden seit Jahrzehnten Polymethylmethacrylat-Knochenzemente zur dauerhaften mechanischen Fixierung von Totalgelenkendoprothesen eingesetzt. Diese basieren auf Pulver-Flüssigkeits-Systemen. Es wurden in jüngster Zeit auch Polymethylmethacrylat-Knochenzemente vorgeschlagen, die auf der Verwendung von Zementpasten beruhen (DE 10 2007 050 762 A1, DE 10 2008 030 312 A1, DE 10 2007 052 116 A1). Für derartige Zemente wurden bisher noch keine geeigneten Kartuschensysteme vorgeschlagen.

Bei der Anwendung von Knochenzementen zur Fixierung von Totalgelenkendoprothesen muss immer berücksichtigt werden, dass bei diesen Operationen das OP-Personal unter zeitlichem Druck steht Bei medizinischen Anwendungen von Kartuschensystemen für die Applikation von pastenförmige Polymethylmethacrylat-Knochenzemente sollten diese daher grundsätzlich so gestaltet sein, dass sie weitgehend resistent gegenüber Anwenderfehlern sind und auch in Stresssituationen schnell und sicher bedient werden können.

Ein essentieller Bestandteil von pastenförmigen Polymethylmethacrylat-Knochenzementen ist das Monomer Methylmethacrylat. Dieses Monomer verdampft leicht und hat bei Raumtemperatur einen recht hohen Dampfdruck. Bei der Verwendung von Methylmethacrylat enthaltenden Pasten muss deshalb unbedingt beachtet werden, dass bei Vakuumeinwirkung, wie bei der Entgasung im Rahmen der Sterilisation mit Ethylenoxid, die Kartuschenkolben in den Kartuschen durch das verdampfende Methylmethacrylat bewegt und im Extremfall aus den Kartuschen ausgestoßen werden können.

Ein Kartuschensystem, das auf der Verpackung von pastösen Mehrkomponentensystemen in Schlauchbeuteln basiert, ist aus der WO 2010/006455 A1 bekannt. Die versiegelten Schlauchbeutel werden dabei in Kartuschen eingebracht. Schlauchbeutel haben den Vorteil, dass sie zur Verpackung von Pasten geeignet sind, die flüchtige Bestandteile enthalten. Schlauchbeutel aus Verbundmaterialien, wie zum Beispiel Aluminiumverbundbeutel, sind dafür besonders geeignet. Die Öffnung der Schlauchbeutel erfolgt durch Schneiden, die sich beim Eindrehen des Austragsrohrs mit drehen. Bei der Drehbewegung der Schneiden werden die Beutel aufgeschnitten und so Öffnungen in den Kartuschen zum Austragen des Inhalts bereitgestellt. Der pastöse Beutelinhalt wird anschließend durch diese Öffnungen in den Kartuschen in Richtung des statischen Mischers gedrückt, bzw. ausgepresst.

Nachteilig ist hieran, dass die Verpackung von pastösen Materialien in Schlauchbeuteln und zusätzlich in Kartuschen sehr kostenintensiv und nur speziellen Anwendungen vorbehalten ist. Für viele Anwendungen, vor allem im medizinischen Bereich, ist es zudem problematisch, dass sich Teile der geschnittenen Schlauchbeutel ablösen können und dann in die pastösen Komponenten gelangen und so das Mischgut kontaminieren können.

Ein gattungsgemäßes Kartuschensystem ist aus der EP 0 431 347 A1 bekannt. Dort sind an den Kartuschenköpfen zweier Kartuschen Öffnungen vorgesehen, durch die der Kartuscheninhalt ausgetrieben werden kann. Ein Verschluss, der in einer Halterung an den Kartuschenköpfen befestigt ist, verschließt die Öffnungen.

Nachteilig ist hieran, dass der Verschluss sowohl in den Öffnungen steckt, als auch in der Halterung eingerastet ist, so dass das Entfernen des Verschlusses aufwendig ist. Zudem kann der vom Kartuschensystem getrennte Verschluss stören und, wenn er zum Wiederverschließen der Öffnungen verwendet werden soll, verloren gegangen sein.

Ferner ist aus DE 297 09 383 U1 ein Drehverschluss bekannt, bei dem eine Austrittsöffnung in einer Umfangswand eines Auslassstutzens gebildet ist. Ein Verschlusselement hat einen parallel zur Umfangswand des Auslassstutzens bewegbaren Abschnitt (Verschlussblende). Die Drehachse des Drehverschlusses steht parallel zur Kartuschenlängsachse.

Aufgabe der Erfindung ist es daher, ein einfaches, kostengünstiges Verschlusssystem für Kartuschensysteme zu entwickeln, das die Probleme der bisherigen Kartuschenverschlusssysteme vermindert beziehungsweise überwindet. Es soll also ein Kartuschenverschlusssystem entwickelt werden, welches Kartuschen sicher verschließt und ein schnelles unkompliziertes Öffnen der einzelnen Kartuschen bei einfacher Bedienbarkeit ermöglicht. Das Anbringen eines Austragsrohrs in die Austragsposition auf den Kartuschenkopf oder Kartuschenköpfen sollte weiterhin mit möglichst wenigen Arbeitsschritten erreichbar sein, um Bedienungsfehler des Anwenders zur vermeiden. Dabei soll eine Verschmutzung des ausgetragenen fließfähigen Materials vermieden werden.

Die Aufgabe wird dadurch gelöst, dass in der Halterung ein Ventil drehbar gelagert ist, wobei das Ventil zumindest einen mit einer Auslassöffnung verbundenen Durchgang durch das Ventil umfasst, das Ventil in einer geschlossenen Position zumindest eine Öffnung wenigstens einer Kartusche, insbesondere alle Öffnungen jeder Kartusche, dicht verschließt, in einer offenen Position des Ventils der Durchgang oder die Durchgänge mit der Öffnung oder den Öffnungen verbunden ist oder sind, so dass der Kartuscheninhalt aus den Kartuschen durch die Auslassöffnung drückbar ist, und wobei das Ventil von der geschlossenen Position in die offene Position durch eine Drehung des Ventils überführbar ist. Am Ventil ist ein Austragsrohr angeordnet, das an der Auslassöffnung beginnt und den Durchgang bis zu einer Austragsrohrspitze verlängert. Weiterhin ist das Austragsrohr bei geschlossenem Ventil parallel zu und zwischen den zumindest zwei Kartuschen angeordnet.

Dabei kann vorgesehen sein, dass jede Kartusche zumindest einen Förderkolben gegenüber des Kartuschenkopfs zum Austreiben des Kartuscheninhalts durch die Öffnung umfasst, der die Kartuschen bodenseitig, insbesondere gasdicht, verschließt.

Ebenso kann vorgesehen sein, dass der Inhalt der Kartuschen ein fließfähiges Material ist.

Auch kann vorgesehen sein, dass an mehreren Öffnungen im Ventil Dichtungsringe angeordnet sind, so dass bei geöffnetem Ventil fluiddichte Verbindungen von den Öffnungen in den Kartuschen zu den Durchgängen bereitgestellt sind.

Weiterhin wird erfindungsgemäß vorgeschlagen, dass am Ventil ein Stutzen angeordnet ist, wobei der Stutzen ein Befestigungsmittel zum Befestigen eines Austragsrohrs umfasst, die Durchgänge sich durch den Stutzen erstrecken und die Auslassöffnung am Stutzen angeordnet ist. Dabei kann vorgesehen sein, dass das Befestigungsmittel ein Gewinde, insbesondere ein Außengewinde ist.

Weiterhin soll zur weiteren Vereinfachung der Bedienbarkeit die Erfindung ein Kartuschensystem mit dem zu entwickelnden Kartuschenverschlusssystem bereitstellen, das eine Bewegung der Kolben bei Einwirkung von Vakuum sicher verhindert. Das Mehrkomponentenkartuschensystem soll weiterhin ermöglichen, dass sich bei Krafteinwirkung in Richtung Kartuschenkopf die Kolben synchron in den Kartuschen bewegen und damit gleichmäßig die fließfähigen Materialien, auspressen, damit das Mischungsverhältnis der fließfähigen Materialien zueinander gewährleistet wird.

Das erfindungsgemäße Kartuschensystem wird daher auch dadurch realisiert, dass parallel zu den Kartuschen, zwischen zumindest zwei Kartuschen, bevorzugt in einem Hohlkörper, um den mehrere Kartuschen parallel angeordnet sind, eine bewegliche Stange parallel zu den Förderkolben angeordnet ist, die mit den Förderkolben über zumindest einen Steg und/oder eine Platte fest verbunden ist, wobei die Stange an der dem Ventil zugewandten Seite ein Rastmittel umfasst und an den Kartuschenwänden, oder bevorzugt an der Innenwand des Hohlkörpers, ein Gegenrastmittel angebracht ist, das mit dem Rastmittel der Stange derart zusammenwirkt, dass eine Bewegung der Stange in Richtung des Kartuschenbodens und damit der Förderkolben aus den Kartuschen heraus deutlich erschwert, insbesondere verhindert ist.

Dabei kann vorgesehen sein, dass die Kartuschenwände zumindest einen Schlitz beginnend am Kartuschenboden aufweisen, der parallel zur Stange angeordnet ist, wobei die Breite des oder der Schlitze zur Aufnahme des oder der Stege ausreicht und insbesondere die Länge des oder der Schlitze bis mindestens zur Hälfte der Kartuschenlänge reicht.

Dabei kann vorgesehen sein, dass das Austragsrohr ein Befestigungsmittel, insbesondere ein Innengewinde, umfasst, mit dem das Austragsrohr am Befestigungsmittel des Stutzens lösbar verbunden ist oder das Austragsrohr fest mit dem Ventil verbunden ist.

Auch kann dabei vorgesehen sein, dass das Austragsrohr einen statischen Mischer umfasst.

Mit der Erfindung wird auch vorgeschlagen, dass das Ventil in der offenen und der geschlossenen Position in Presspassung über den Öffnungen an den Kartuschenköpfen angeordnet ist und diese dicht abschließt oder dicht mit den Durchgängen verbindet. Weiterhin wird erfindungsgemäß vorgeschlagen, dass das Kartuschensystem zumindest zwei parallel zueinander angeordnete Kartuschen zum Mischen und Applizieren eines Mischguts, insbesondere eines medizinischen Zements, umfasst, vorzugsweise drei parallel zueinander angeordnete Kartuschen.

Dabei kann vorgesehen sein, dass die Durchgänge im Ventil die Öffnungen von zumindest zwei Kartuschen mit der Auslassöffnung verbinden, wenn das Ventil in der geöffneten Position ist. Auch wird vorgeschlagen, dass das Ventil zylindrisch geformt ist, die Form eines Zylinders mit elliptischer Grundfläche aufweist oder die Form eines Zylinderabschnitts hat.

Es kann auch erfindungsgemäß vorgesehen sein, dass der drehbare Zylinder (20, 120, 220, 320, 420, 520) an beiden Schmalseiten verschlossen ist und sich vorzugsweise konisch von einer Schmalseite zur anderen verjüngt.

Ferner wird vorgeschlagen, dass das Austragsrohr unmittelbar oberhalb der Verbindungsstelle zwischen dem als drehbaren Zylinder ausgebildeten Ventil und dem Austragsrohr einen Außendurchmesser vorzugsweise von gleich oder kleiner dem Abstand zwischen den jochförmigen Lagern, die die Halterung bilden, hat

Es kann erfindungsgemäß auch vorgesehen sein, dass ein Lager einen größeren Innendurchmesser als das zweite Lager hat und dass die Verbindungsstelle vom drehbaren Zylinder zum Austragsrohr zum ersten Lager sich in einem solchen Abstand befindet, der kleiner ist als der halbe Außendurchmesser des Austragsrohrs unmittelbar oberhalb der Verbindungsstelle des drehbaren Zylinders mit dem Austragsrohr.

Mit der Erfindung wird zudem vorgeschlagen, dass Dichtungsringe am Ventil angeordnet sind, die die Öffnungen in den Kartuschen bei geschlossener und/oder offener Position des Ventils abdichten.

Unter dem Begriff fließfähiges Material werden im Sinne der vorliegenden Erfindung flüssige Materialien, zähflüssige Materialien und auch hochviskose Materialien, die nur bei Druckbeaufschlagung fließen, verstanden.

Im Folgenden werden Ausführungsbeispiele der Erfindung anhand von neun schematisch dargestellten Zeichnungen erläutert. Dabei zeigt:
- Figur 1:: eine Querschnittansicht in Längsrichtung eines Kartuschensystems;
- Figur 2:: eine Querschnittansicht in Längsrichtung eines zweiten erfindungsgemäßen Kartuschensystems mit geöffnetem Verschluss;
- Figur 3:: eine Querschnittansicht in Längsrichtung des zweiten erfindungsgemäßen Kartuschensystems nach Figur 2 mit geschlossenem Verschluss;
- Figur 4:: eine Querschnittansicht in Längsrichtung eines dritten erfindungsgemäßen Kartuschensystems mit geschlossenem Verschluss und Austragsrohr,
- Figur 5:: eine Querschnittansicht in Längsrichtung des dritten erfindungsgemäßen Kartuschensystems nach Figur 4 mit geöffnetem Verschluss und Austragsrohr,
- Figur 6:: eine Querschnittansicht in Längsrichtung eines vierten erfindungsgemäßen Kartuschensystems mit geöffnetem Verschluss und Arretiervorrichtung;
- Figur 7a):: eine Seitenaufsicht des dritten erfindungsgemäßen Kartuschensystems nach Figur 4 mit geschlossenem Verschluss und Austragsrohr,
- Figur 7b):: eine Seitenaufsicht des dritten erfindungsgemäßen Kartuschensystems nach Figur 5 mit geöffnetem Verschluss und Austragsrohr,
- Figur 8:: eine Querschnittansicht in Querrichtung eines erfindungsgemäßen Verschlusses; und
- Figur 9:: eine Querschnittansicht in Längsrichtung eines vierten erfindungsgemäßen Kartuschensystems mit geöffnetem Verschluss.

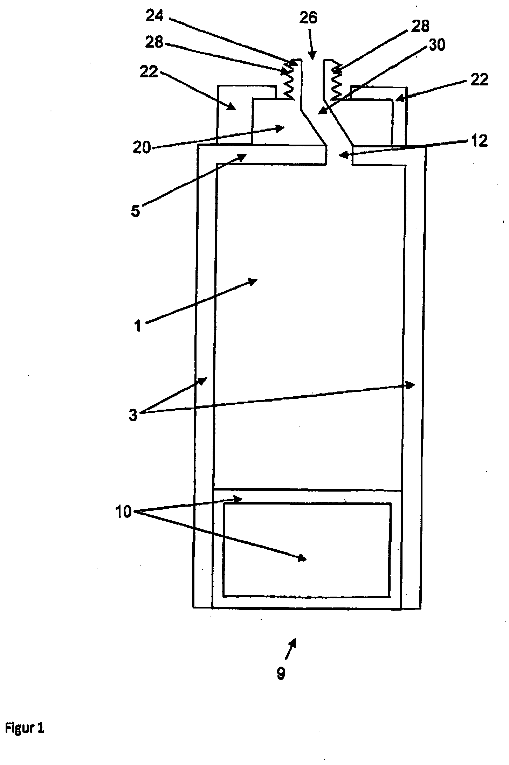

Die in Figur 1 dargestellte schematische Querschnittansicht in Längsrichtung zeigt eine Kartusche (1), die auf allen Seiten durch Kartuschenwände (3), an der Vorderseite durch einen Kartuschenkopf (5) und aus Richtung des Kartuschenbodens (9) durch einen Förderkolben (10) begrenzt ist. In der Wand, die den Kartuschenkopf (5) bildet, ist eine Öffnung (12) vorgesehen, durch die ein in der Kartusche (1) enthaltenes fließfähiges Material (nicht gezeigt) durch Einschieben der Förderkolben (10) ausgetrieben werden kann.

Am Kartuschenkopf (5) ist ein zylindrisches Ventil (20) in einer Halterung (22) angeordnet, das um seine Symmetrieachse drehbar in der Halterung (22) gelagert ist Der Kartuschenkopf (5) umfasst eine Vertiefung (nicht gezeigt) der Form eines Zylindermantelabschnitts, so dass das zylindrische Ventil (20) genau in die Vertiefung passt. Die Öffnung (12) nicht innerhalb dieser Vertiefung. Das zylindrische Ventil (20) umfasst auf einer Seite seiner Zylindermantelfläche einen hohlen, zylindrischen Stutzen (24) mit einer Auslassöffnung (26), dessen Symmetrieachse senkrecht zur Symmetrieachse des Ventils (20) angeordnet ist. Auf der Außenfläche des Stutzens (24) ist ein Gewinde (28) als Befestigungsmittel für ein Austragsrohr (nicht gezeigt) angeordnet.

Im Inneren des ansonsten massiven zylindrischen Ventils (20) befindet sich ein Durchgang (30), der die der Auslassöffnung (26) gegenüberliegende Öffnung des Stutzens (24) durch das zylindrische Ventil (20) fortsetzt, bis auf die dem Stutzen (24) gegenüberliegende Seite des Ventils (20). Die beiden im Ventil (20) vorgesehenen Öffnungen sind versetzt zu einander angeordnet. Dadurch wird erreicht, dass im Kartuschenkopf (5) eine Vertiefung (nicht gezeigt) für den Stutzen (24) vorgesehen sein kann, in die der Stutzen (24) bei einer Drehung des Ventils (20) versenkt werden kann, ohne die Öffnung (12) i Kartuschenkopf (5) zu beeinträchtigen.

Es besteht eine durchgehende Öffnung von der Kartusche (1) über die Öffnung (12) und den Durchgang (30) bis zur Auslassöffnung (26), so dass das fließfähige Material aus dem Inneren der Kartuschen (1) durch die durchgehende Öffnung aus dem Kartuschensystem ausgetrieben werden kann. Der Verschluss des Kartuschensystems, der durch das um seine Symmetrieachse drehbar gelagerte Ventil (20) gebildet wird, ist also offen.

Die Halterung (22) kann aus zwei Lagern (22) bestehen, die nicht gleich aufgebaut sein müssen. Eine asymmetrische Form der Lager (22) hat den Vorteil, dass das Ventil (20) nur in einer bestimmten Orientierung in die Halterung (22) einbaubar ist.

Das Ventil (20) erstreckt sich durch die Halterung (22) hindurch, wobei der Stutzen (24) nicht zwischen den beiden Lagern (20) der Halterung (22) am Ventil (20) abgeordnet ist, sondern außerhalb. Bei einer Drehung des Ventils (20) um 180° um seine Symmetrieachse befindet sich der Stutzen (24) bei ausreichendem Abstand von der Halterung (22) dann seitlich neben der Kartusche (1). Es ist vorgesehen, dass ein Austragsrohr
(nicht gezeigt), das mit den Stutzen (24) verbunden ist, bei dieser geschlossenen Position des Ventils (20) neben der Kartusche (1) angeordnet ist, wodurch eine kompakte Bauweise des Kartuschensystems erreicht wird.

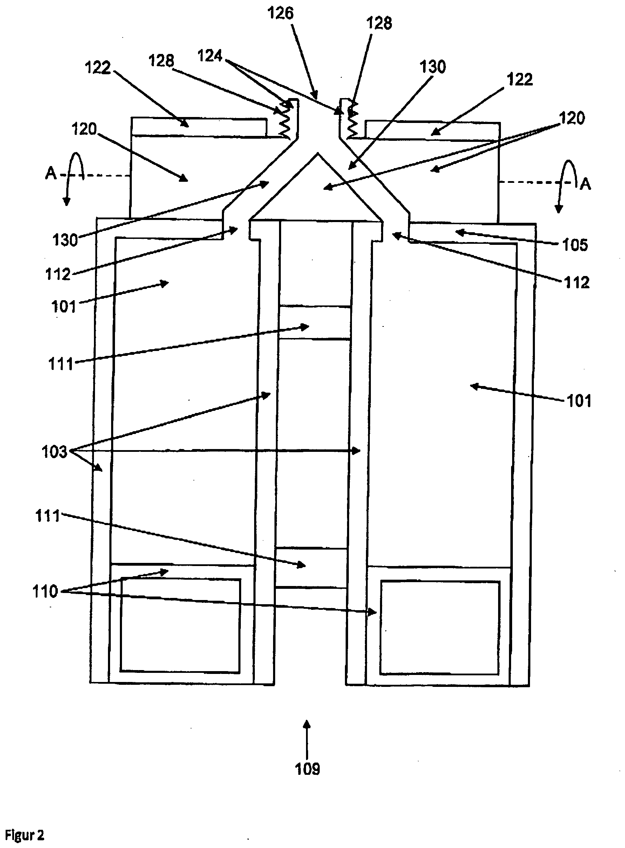

Figur 2 zeigt eine schematisch dargestellte Querschnittansicht eines zweiten erfindungsgemäßen Kartuschensystems, das zum Mischen eines Mischguts bestehend aus zwei oder mehr Komponenten geeignet ist. Dazu umfasst das Kartuschensystem zumindest zwei Kartuschen (101), in denen die aus fließfähigen Materialien bestehenden Ausgangskomponenten des Mischguts enthalten sind. Neben den zwei in Figur 2 dargestellten Kartuschen (101) können auch weitere Kartuschen vorgesehen sein, die beispielsweise hinter den beiden dargestellten Kartuschen (101) angeordnet sind. Die Kartuschen (101) sind durch Kartuschenwände (103) seitlich und durch Kartuschenköpfe (105) auf der Vorderseite begrenzt. Aus Richtung des Kartuschenbodens (109) sind die Kartuschen (101) durch Förderkolben (110) gasdicht verschlossen, so dass der Kartuscheninhalt durch Beaufschlagung der Förderkolben (110) mit Druckluft aus den Kartuschen (101) drückbar ist. Die beiden Kartuschen (101) sind über Stege (111) fest miteinander verbunden. In den Kartuschenköpfen (105) sind Öffnungen (112) vorgesehen.

Über den beiden Öffnungen (112) ist an den Kartuschenköpfen (105) ein Ventil (120) angeordnet, das drehbar um die Drehachse (A) in einer Halterung (122), die fest mit den Kartuschenköpfen (105) verbunden ist, gelagert ist. Ein zylindrischer Stutzen (124), der als Hohlkörper ausgebildet ist und der eine Auslassöffnung (126) und ein Außengewinde (128) umfasst, ist auf einer Seite des Ventils (120) angeordnet. Die Halterung (122) weist in diesem Bereich eine Auslassung auf. Dazu kann die Halterung (122) aus zwei Teilen aufgebaut sein. Das Außengewinde (128) ist zum Befestigen eines Austragsrohrs (nicht gezeigt) mit einem passenden Innengewinde geeignet.

Im Inneren des Ventils (120) befinden sich zwei Durchgängen (130), die in der in Figur 2 dargestellten geöffneten Position des Ventils (120) eine durchgehende Verbindung von den beiden Öffnungen (112) der Kartuschen (101) zur Auslassöffnung (126) bilden. Wird ein Druck auf die Förderkolben (110) ausgeübt, so wird das in den Kartuschen (101) enthaltene fließfähige Material (nicht gezeigt) aus diesen durch die Öffnungen (112) und die Durchgänge (130) in den Stutzen (124) gedrückt, wo die beiden Ausgangskomponenten zu einem Mischgut gemischt werden. Auf der Auslassöffnung (126) ist ein Austragsrohr (nicht gezeigt) angebracht, das einen statischen Mischer enthält, so dass die beiden Ausgangskomponenten im Austragsrohr besser durchmischt werden. Das Mischgut kann dann über eine Austragsrohrspitze des Austragsrohrs appliziert werden.

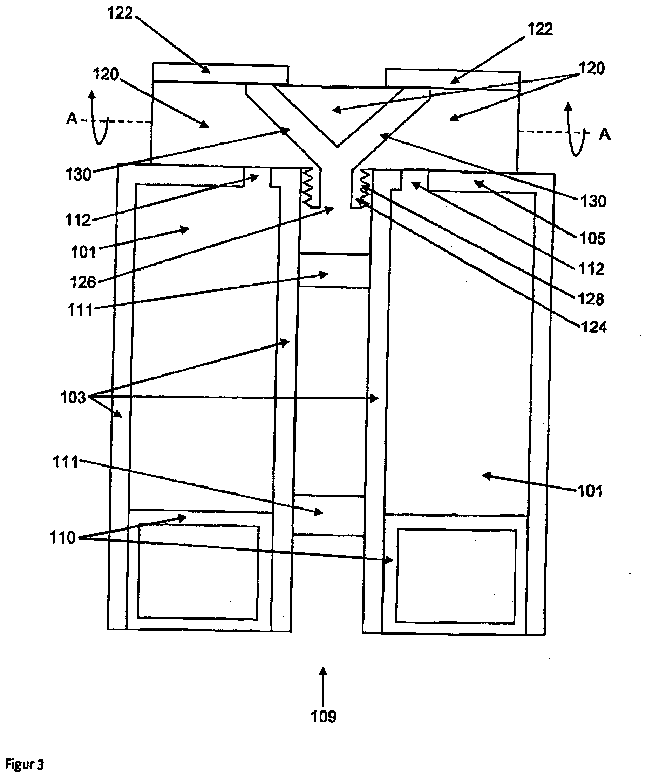

Die Öffnungen (112) und damit das Kartuschensystem kann durch eine Drehung des Ventils (120) um die Drehachse (A) geschlossen werden. Eine solche Anordnung ist in Figur 3 in schematischer Querschnittansicht dargestellt. Durch Drehung des Ventils (120) um 180 ° sind die beiden Öffnungen (112) der Kartuschen (101) verschlossen. Der Stutzen (124) mit der Auslassöffnung (126) und dem Gewinde (128) ist in den Zwischenraum zwischen den beiden Kartuschen (101), der durch die ihm zugewandten Kartuschenwände (103) begrenzt ist, positioniert. Dazu ist der Abstand zwischen den beiden Kartuschen (101) groß genug, um die volle Breite des Stutzens (124), an den ein Austragsrohr (nicht dargestellt) installiert ist, aufzunehmen. Das Ventil (120) sitzt in Presspassung auf dem die Öffnungen (112) umgebenden Bereichen der Kartuschenköpfe (105). Dazu ist die äußere Form des Ventilsitzes an den Kartuschenköpfen (105) an die äußere Form des Ventils (120) angepasst.

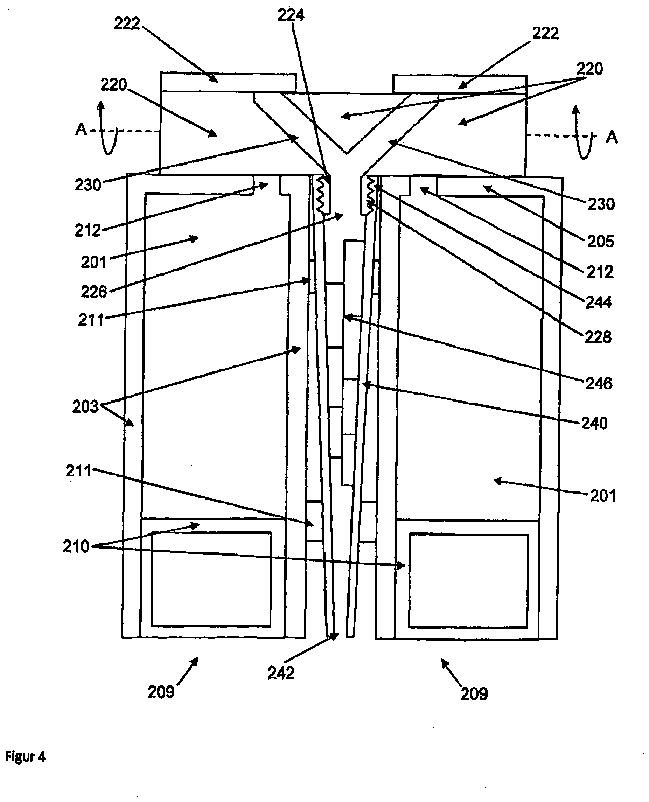

Eine solche Konstellation ist für ein drittes Ausführungsbeispiel als schematische Skizze in Figur 4 gezeigt. Zwischen zwei Kartuschen (201), die über Stege (211) miteinander verbunden sind, ist ausreichend Platz für die volle Breite eines Austragsrohrs (240). Die Kartuschen (201) sind an den Seiten durch Kartuschenwände (203), ihrer Oberseite durch Kartuschenköpfe (205) und ihrer Unterseite durch Förderkolben (210) geschlossen. Die Förderkolben (210) sind aus Richtung der Kartuschenböden (209) in die Kartuschen (201) einschiebbar. In den Kartuschenköpfen (205) befinden sich Öffnungen (212) für in den Kartuschen (201) gelagerte, fließfähige Materialien.

In einer Halterung (222) ist ein Ventil (220) in Form eines Zylinders, drehbar um die Symmetrieachse (A) des Zylinders als Drehachse (A) gelagert. Auf einer Zylindermantelfläche des Ventils (220) befindet sich ein Stutzen (224) mit einem Gewinde (228). Der Stutzen (224) ist nach oben offen und bildet dort eine Auslassöffnung (226). Durchgänge (230) sind im Ventil (220) angeordnet, die zwei Öffnungen auf der dem Stutzen (224) gegenüberliegenden Seite mit der Auslassöffnung (226) verbinden. Die beiden Öffnungen (212) sind durch die Zylindermantelwände des Ventils (220) geschlossen. Wenn das geschlossene Ventil (220) um 180° um die Drehachse (A) gedreht wird, so liegen die beiden Öffnungen im Ventil (220) über den Öffnungen (212) der Kartuschen (201). Dann ist das Kartuschensystem, beziehungsweise der Verschluss, d. h. das Ventil (220) geöffnet.

Das Austragsrohr (240) ist innen hohl und mündet in eine Austragsrohrspitze (242). Auf der gegenüberliegenden Seite umfasst das Austragsrohr (240) ein Befestigungsmittel (244) in Form eines Innengewindes. Das Innengewinde (244) des Austragsrohrs (240) ist auf das Außengewinde (228) des Stutzens (224) aufgeschraubt. Alternativ dazu kann das Austragsrohr (240) auch fest mit dem Stutzen (224) verbunden sein. Im Inneren des Austragsrohrs (240) ist ein statischer Mischer (246) angeordnet.

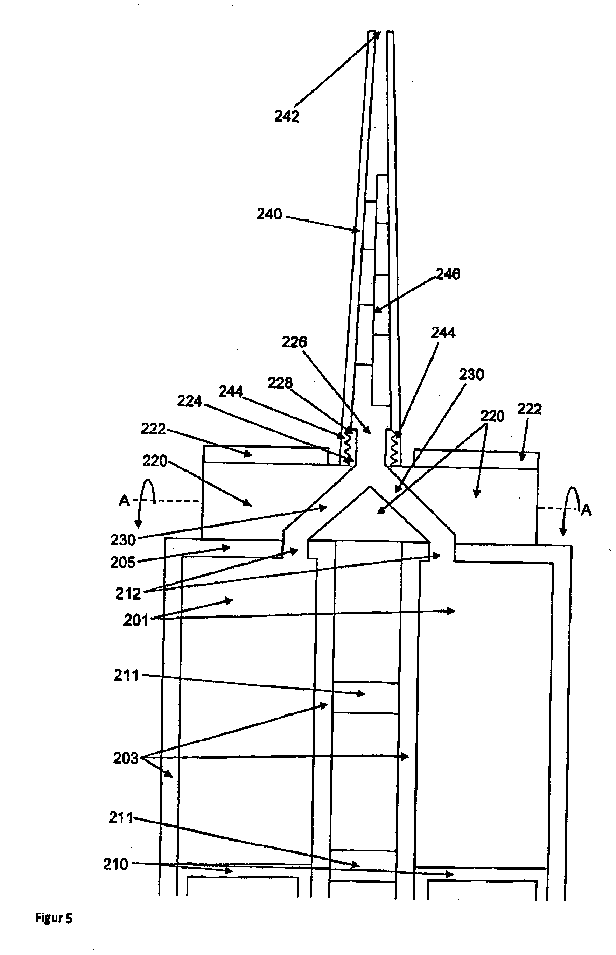

Die Stege (211) müssen eine Wölbung (in die Zeichenebene nach hinten) aufweisen, damit das Austragsrohr (240) genau zwischen den beiden Kartuschen (201) positioniert werden kann. Auch dürfen die beiden Stege (211) nicht die Kippbewegung des Austragsrohrs (240) aufgrund einer Drehung des Ventils (220) behindern. Wenn das Ventil (220) um die Drehachse (A) gedreht wird, so wird der Verschluss des Kartuschensystems vom geschlossenen in den offenen Zustand überführt Gleichzeitig wird auch das Austragsrohr (240) um 180° geschwenkt Dieser offene Zustand des Kartuschensystems ist als schematischer Längsschnitt in Figur 5 dargestellt. Die beiden gegenüber dem Stutzen (224) liegenden Öffnungen des Ventils (220) liegen im geöffneten Zustand des Ventils (220) über den Öffnungen (212) der Kartuschen (201). Dadurch ist über die Durchgänge (230) eine Verbindung zwischen den Öffnungen (212) und den Inneren des Austragsrohrs (240) hergestellt.

Wird also von außen, zum Beispiel durch Druckluft, eine Kraft auf die Förderkolben (210) ausgeübt, so wird der Inhalt der Kartuschen (201) durch die Öffnungen (212) über die Durchgänge (230) und die Auslassöffnung (226) in das Austragsrohr (240) befördert Mit Hilfe des statischen Mischers (246) werden dort die Inhalte der Kartuschen (201) durchmischt und das so entstandene Mischgut wird aus der Austragsrohrspitze (242) heraus gepresst

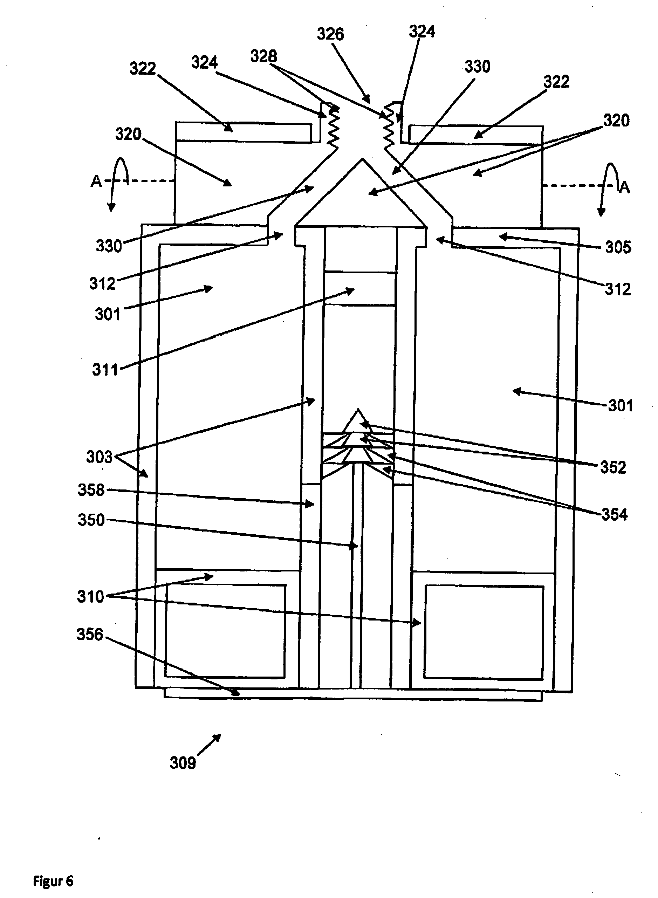

Figur 6 zeigt die schematische Darstellung eines vierten erfindungsgemäßen Ausführungsbeispiels als Querschnittdarstellung. Zwei Kartuschen (301), die von Kartuschenwänden (303), einem Kartuschenkopf (305) auf der Vorderseite und Förderkolben (310) auf der Seite des Kartuschenbodens (309), begrenzt sind, haben jeweils eine Öffnung (312) im Kartuschenkopf (305). Die beiden Kartuschen (301) sind über einen Steg (311) miteinander verbunden. An den Kartuschenköpfen (305) ist in einer Halterung (322) ein zylindrisches Ventil (320) um die Drehachse (A) drehbar gelagert. Innerhalb des Ventils (320) befinden sich Durchgänge (330), die die Öffnungen (312) mit einer auf der gegenüberliegenden Seite des Ventils (320) angeordneten Auslassöffnung (326) verbinden. Im Bereich der Auslassöffnung (326) ist ein Stutzen (324) am Ventil (320) angeordnet. Der Stutzen (324) ist ein Hohlkörper, auf dessen Innenseite ein Befestigungsmitteln (328) in Form eines Innengewindes oder eines Rastmittels angeordnet ist

Im Bereich zwischen den beiden Kartuschen (301) ist eine Stange (350) angeordnet, an deren Spitze ein Rastmittel (352) vorgesehen ist. Das Rastmittel (352) kann In ein Gegenrastmittel (354) greifen, das an den zum Zwischenraum gewandten Kartuschenaußenwänden (303) der Kartuschen (301) angeordnet ist. Sobald die Rastmittel (352) in die Gegenrastmittel (354) greifen, wird eine Bewegung der Stange (350) in Richtung des Kartuschenbodens (309) unterbunden. Am Kartuschenboden (309) ist ein Steg (356) angeordnet, der die beiden Förderkolben (310) und die Stange (350) fest miteinander verbindet. Der Zwischenraum kann auch als zumindest bereichsweise geschlossener Hohlkörper aufgebaut sein, um den herum die Kartuschen (301) angeordnet sind.

Aufgrund des Stegs (356) ist nur eine synchrone Bewegung der Förderkolben (310) und der Stange (350) möglich. Der Steg (356) kann durch einen Schlitz (358) durch die Kartuschenwände (303) hindurch bewegt werden, damit Förderkolben (310) weit ins Innere der Kartuschen (301) geschoben werden können. Alternativ dazu könnte der Steg (356) auch als Doppelbogen ausgestaltet sein, beziehungsweise über Stangen mit den Förderkolben (310) und der Stange (350) verbunden sein, ohne dass Schlitze (358) in den Kartuschenwänden (303) notwendig wären.

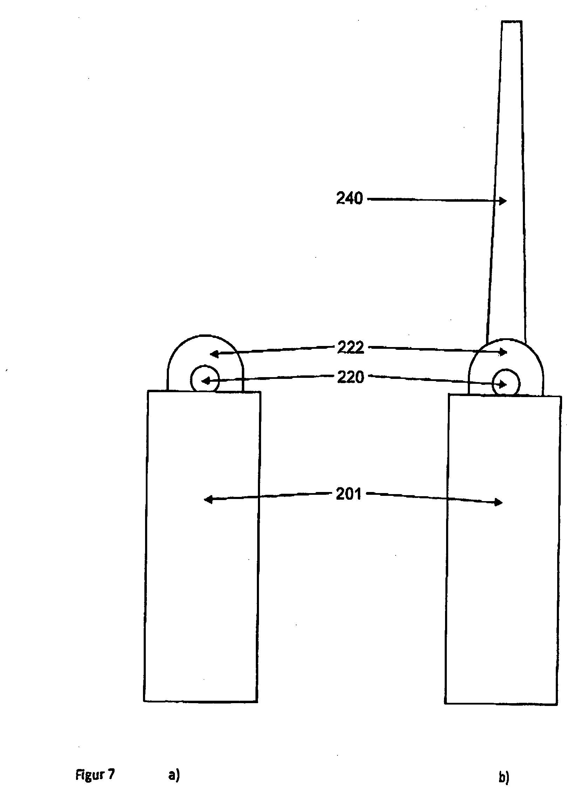

Figur 7a) zeigt ein geschlossenes Kartuschensystem mit eingeklapptem Austragsrohr nach Figur 4 als Aufsicht auf die Seite des Kartuschensystems. Oberhalb der Kartusche (201) ist das drehbare Ventil (220) in der Halterung (222) angeordnet Das Austragsrohr ist hier nicht zu sehen, da es sich zwischen den Kartuschen (201) befindet

Figur 7b) zeigt ein geöffnetes Kartuschensystem mit ausgeklapptem Austragsrohr (240) nach Figur 5 als Aufsicht auf die Seite des Kartuschensystems. Das Austragsrohr (240) ist oberhalb der Halterung (222) für das drehbare Ventil (220) zu erkennen. Das drehbare Ventil (220) ist über die Halterung (222) fest mit der Kartusche (201) verbunden.

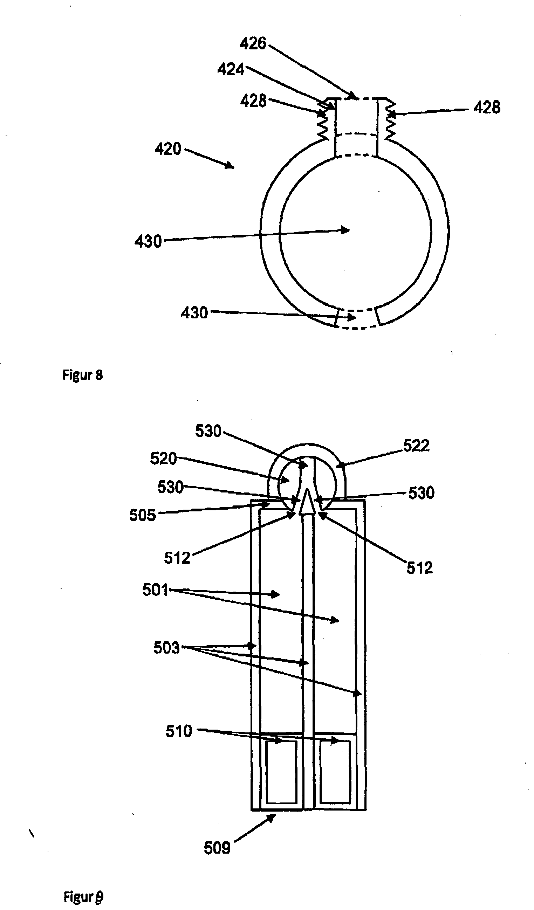

Figur 8 zeigt schematisch ein drehbar zu lagerndes Ventil (420) für einen erfindungsgemäßen Verschluss für ein erfindungsgemäßes Kartuschensystem als Querschnittansicht. Das Ventil (420) umfasst auf seiner Oberseite einen hohlen Stutzen (424) mit einem Außengewinde (428). Der Stutzen (424) mündet in eine Auslassöffnung (426). Auf der Unterseite des Ventils (420) ist eine Öffnung vorgesehen. Das gesamte Ventil (420) ist innen hohl. Der Hohlraum im Inneren des Ventils (420) und die untere Öffnung bilden gemeinsam einen Durchgang (430) durch das Ventil (420).

Figur 9 zeigt ein fünftes Ausführungsbeispiel eines erfindungsgemäßen Kartuschensystems in Querschnittansicht. Zwei Kartuschen (501), die seitlich durch Kartuschenwände (503), oben durch Kartuschenköpfe (505) und unten durch gasdichte Förderkolben (510) geschlossen sind, umfassen Öffnungen (512) in den Kartuschenköpfen (505). Im Presssitz mit den Kartuschenköpfen (505) befindet sich ein zylindrisches Ventil (520), das drehbar in einer Halterung (522) an den Kartuschenköpfen (505) gelagert ist. Im Inneren des Ventils (520) befinden sich Durchgänge (530), die bei einer geöffneten Position des Ventils (520) einen Durchlass von den Öffnungen (512) zu einer gegenüber den Kartuschenköpfen (505) im Ventil (520) angeordneten Auslassöffnung (nicht gezeigt) bilden. Wird aus Richtung des Kartuschenbodens (509) ein Druck auf die Förderkolben (510) ausgeübt, so kann der Inhalt der Kartuschen (501) durch das Ventil (520) hindurch aus dem Kartuschensystem gepresst werden.

Durch eine Drehung des Ventils (520) lässt sich ein vollständiges Verschließen der Öffnungen (512) und damit des Kartuschensystems erreichen. Bei der Anordnung des fünften Ausführungsbeispiels ist das Ventil (520) im Vergleich zu den anderen Ausführungsbeispielen senkrecht zu seiner Symmetrieachse gedreht orientiert. Hinter den beiden Kartuschen (501) ist noch eine weitere Kartusche mit einer weiteren Öffnung (nicht gezeigt) angeordnet. Ein weiterer Durchgang (nicht gezeigt) erstreckt sich von dieser weiteren Öffnung durch das Ventil (520) zur Auslassöffnung. Dadurch sind drei Kartuschen mit der Auslassöffnung verbunden, so dass mit einem solchen Kartuschensystem ein Mischgut aus drei Komponenten herstellbar ist.

Die Ausführungsbespiele lassen sich ohne weiteres auf ein Kartuschensystem umfassend drei, vier, fünf oder mehr Kartuschen verallgemeinern. Wenn die Ausgangskomponenten in einem anderen Mischungsverhältnis als in gleichen Anteilen beigemischt werden müssen, um das gewünschte Mischgut zu erhalten, kann das Verhältnis der Querschnittsflächen der Kartuschen (1, 101, 201, 301, 501) an das Mischungsverhältnis angepasst werden. Werden die Förderkolben (10, 110, 210, 310, 510) dann mit gleicher Geschwindigkeit vorgetrieben so ergibt sich eine Mischung mit dem gewünschten Mischungsverhältnis. Der Vortrieb mit gleicher Geschwindigkeit kann sichergestellt werden, indem alle Förderkolben (10, 110, 210, 310, 610) mit einem oder mehreren Stegen (111, 211, 311) fest miteinander verbunden sind. Alternativ kann ein bestimmtes Mischungsverhältnis auch dadurch eingestellt werden, indem eine Übersetzung oder ein geeignetes Getriebe die verschiedenen Förderkolben (10, 110, 210, 310, 510) mit unterschiedlichen Geschwindigkeiten vortreibt. Durch unterschiedliche Veränderung der Vortriebsgeschwindigkeit der verschiedenen Förderkolben (10, 110, 210, 310, 510) kann das Mischungsverhältnis der Ausgangskomponenten während des Austreibens verändert werden und so unterschiedliche physikalische Eigenschaften des ausgepressten Mischguts erzeugt werden. Dadurch entstehen Eigenschaftsgradienten in dem erzeugten Mischgut. Für das Beispiel eines Zements oder Klebers können so Verbundwerkstoffe als Verbindungen aufgebaut werden, die eine bessere Haltbarkeit aufweisen, als homogene Verbindungen.

Ein erfindungsgemäßes Kartuschensystem kann also dadurch charakterisiert sein, dass
a) ein Austragsrohr (240) angeordnet ist,
b) dass das Austragsrohr (240) mit einem Ende mit einem drehbaren Zylinder (20, 120, 220, 320, 420, 520) verbunden ist,
c) der drehbare Zylinder (20, 120, 220, 320, 420, 520) mindestens zwei miteinander verbundene Öffnungen besitzt,
d) wobei mindestens eine Öffnung im Zylinder (20, 120, 220, 320, 420, 520) durchgängig mit dem Austragsrohr (240) verbunden ist,
e) dass der drehbare Zylinder (20, 120, 220, 320, 420, 520) in einer Halterung (22, 122, 222, 322, 522) umfassend mindestens zwei jochförmige Lager (22, 122, 222, 322, 522) gelagert ist, die mit mehreren Kartuschenköpfen (5, 105, 205, 305, 505) verbunden sind,
f) dass mindestens eine Öffnung (12, 112, 212, 312, 512) in den Kartuschenköpfen (5, 105, 205, 305, 505) vorhanden ist, die mit den Innenräumen der Kartuschen (1, 101, 201, 301, 501) durchlässig verbunden ist,
g) dass das mit dem drehbaren Zylinder (20, 120, 220, 320, 420, 520) verbundene Austragsrohr (240) zwischen den jochförmigen Lagern (22, 122, 222, 322, 522) angeordnet ist,
h) dass der Zylinder (20, 120, 220, 320, 420, 520) um seine Zylinderachse um 180 ° drehbar angeordnet ist, und
i) dass der drehbare Zylinder (20, 120, 220, 320, 420, 520) mit den Öffnungen des Zylinders (20, 120, 220, 320, 420, 520) und den Öffnungen (12, 112, 212, 312, 512) der Kartuschen (1, 101, 201, 301, 501) sowie mit den jochförmigen Lagern (22, 122, 222, 322, 522) mindestens ein Ventil (20, 120, 220, 320, 420, 520) bildet.

Es kann auch erfindungsgemäß vorgesehen sein, dass der drehbare Zylinder (20, 120, 220, 320, 420, 520) an beiden Schmalseiten verschlossen ist und sich vorzugsweise konisch von einer Schmalseite zur anderen verjüngt.

Ferner wird vorgeschlagen, dass das Austragsrohr (240) unmittelbar oberhalb der Verbindungsstelle zwischen dem drehbaren Zylinder (20, 120, 220, 320, 420, 520) und dem Austragsrohr (240) einen Außendurchmesser vorzugsweise von gleich oder kleiner dem Abstand zwischen den jochförmigen Lagern (22, 122, 222, 322, 522) hat.

Es kann erfindungsgemäß auch vorgesehen sein, dass ein Lager (22, 122, 222, 322, 522) einen größeren Innendurchmesser als das zweite Lager (22, 122, 222, 322, 522) hat und dass die Verbindungsstelle vom drehbaren Zylinder (20, 120, 220, 320, 420, 520) zum Austragsrohr (240) zum ersten Lager (22, 122, 222, 322, 522) sich in einem solchen Abstand befindet, der kleiner ist als der halbe Außendurchmesser des Austragsrohrs (240) unmittelbar oberhalb der Verbindungsstelle des drehbaren Zylinders (20, 120, 220, 320, 420, 520) mit dem Austragsrohr (240).

Das Austragsrohr (240) ist mit den Kartuschen (1, 101, 201, 301, 501) über den drehbaren Zylinder (20, 120, 220, 320, 420, 520), der sich in mindesten zwei jochförmigen Lagern (22, 122, 222, 322, 522) der Halterung (22, 122, 222, 322, 522) befindet, verbunden. Das erfindungsgemäße Kartuschenverschlusssystem funktioniert in der Weise, dass im verschlossenen Zustand das Austragsrohr (240) in Richtung Kartuschenboden (9, 109, 209, 309, 509) gedreht ist. Das Austragsrohr (240) liegt parallel zu den Kartuschen (1, 101, 201, 301, 501). Zum Öffnen wird das nach unten in Richtung Kartuschenboden (9, 109, 209, 309, 509) geklappte Austragsrohr (240) einfach nach oben in Richtung Kartuschenkopf (5, 105, 205, 305, 505) oder Kartuschenköpfe (5, 105, 205, 305, 505) gedreht. Wenn das Austragsrohr (240) nach unten in Richtung Kartuschenboden (9, 109, 209, 309, 509) gedreht ist, dann befinden sich die Öffnungen im Zylinder (20, 120, 220, 320, 420, 520) nicht deckungsgleich zu den Öffnungen (12, 112, 212, 312, 512) der Kartuschenköpfe (5, 105, 205, 305, 505). Durch Drehung des Austragsrohrs (240) nach oben wird der drehbare Zylinder (20, 120, 220, 320, 420, 520) so gedreht, dass die Öffnungen im Zylinder (20, 120, 220, 320, 420, 520) deckungsgleich zu den Öffnungen (12, 112, 212, 312, 512) im Kartuschenkopf (5, 105, 205, 305, 505) sind.

Das bedeutet, der Anwender braucht nur das Austragsrohr (240) nach oben in Richtung des Kartuschenkopfs (5, 105, 205, 305, 505) in die Applikationsposition zu klappen, um das Ventil (20, 120, 220, 320, 420, 520) und damit die Kartuschen (1, 101, 201, 301, 501) zu öffnen. Der Anwender muss das Austragsrohr (240) nicht mit den Kartuschen (1, 101, 201, 301, 501) verbinden. Dadurch ist ein Montagefehler bei der Applikation von vornherein ausgeschlossen. Bei der Verwendung von Mehrkomponentenkartuschen werden alle Kartuschen (1, 101, 201, 301, 501) synchron durch die Drehung des Austragsrohrs (240) und damit des drehbaren Zylinders (20, 120, 220, 320, 420, 520) geöffnet. Vorteilhaft ist weiterhin, dass das Austragsrohr (240) mit den Kartuschen (1, 101, 201, 301, 501) verbunden ist. Dadurch kann das Austragsrohr (240) vor der Applikation beim Auspacken aus den Packmitteln nicht verloren gehen. Weiterhin wird kein separates Packmittel für das Austragsrohr (240) benötigt.

Dem Anwender steht mit dem erfindungsgemäßen Kartuschenverschlusssystem ein System zur Verfügung, das schnell, einfach und sicher bedient werden kann.

An den Lagern (22, 122, 222, 322, 522) können, als vorteilhafte Ausgestaltung der Erfindung, in der Kunststofftechnik übliche Rastvorrichtungen zum Beispiel in Form von in einer Richtung beweglicher Zapfen angeordnet sein, die das Austragsrohr (240) in der Applikationsstellung fixieren und ein Zurückdrehen des Austragsrohrs (240) aus der Applikationsstellung verhindern. Eine weitere vorteilhafte Ausgestaltung kann sein, dass an der Unterseite des drehbaren Zylinders (20, 120, 220, 320, 420, 520) in der Kunststofftechnik übliche Rastvorrichtungen angebracht sind, die ein Zurückdrehen des Austragsrohrs (240) aus der Applikationsstellung verhindern.

Es ist erfindungsgemäß, dass der drehbare Zylinder (20, 120, 220, 320, 420, 520) an beiden Schmalseiten verschlossen ist und sich vorzugsweise konisch von einer Schmalseite zur anderen verjüngt. Dadurch ist es möglich den drehbaren Zylinder (20,120,220,320,420, 520) problemlos in den Lagern (22, 122, 222, 322, 522) zu montieren.

Es kann vorgesehen sein, dass der Zylinder (20, 120, 220, 320, 420, 520) sich in Presspassung in den jochförmigen Lagern (22, 122, 222, 322, 522) befindet. Durch die Presspassung wird eine ausreichende Dichtwirkung erreicht. Es ist auch im Rahmen der Erfindung, dass zusätzlich Dichtungsringe am drehbaren Zylinder (20, 120, 220, 320, 420, 520) angeordnet sein können, falls die in den Kartuschen (1, 101, 201, 301, 501) zu lagernden fließfähigen Materialien auf Grund ihrer Eigenschaften dies erfordern sollten. Sofern nur eine der Ausgangskomponenten für ein Mischgut derart dünnflüssig ist, dass die Verbindung ohne Dichtungsring nicht ausreichend dicht wäre, reicht es aus, dass der zugehörigen Öffnung (12, 112, 212, 312, 512) am Ventil (20, 120, 220, 320, 420, 520) zusätzlich je ein Dichtungsring für die geschlossene und die offene Position zugeordnet ist.

Erfindungsgemäß kann auch vorgesehen sein, dass das Austragsrohr (240) unmittelbar oberhalb der Verbindungsstelle zwischen dem drehbaren Zylinder (20, 120, 220, 320, 420, 520) und dem Austragsrohr (240) einen Außendurchmesser vorzugsweise von gleich oder kleiner dem Abstand zwischen den jochförmigen Lagern (22, 122, 222, 322, 522) hat. Das Austragsrohr (240) fixiert den Zylinder (20, 120, 220, 320, 420, 520) zwischen den Lagern (22, 122, 222, 322, 522). Dadurch kann der Zylinder (20, 120, 220, 320, 420, 520) nicht heraus rutschen.

Vorteilhaft ist, dass ein erstes Lager (22, 122, 222, 322, 522) einen größeren Innendurchmesser als das zweite Lager (22, 122, 222, 322, 522) hat und dass die Mitte der Verbindungsstelle vom drehbaren Zylinder (20, 120, 220, 320, 420, 520) zum Austragsrohr (240) zum ersten Lager (22, 122, 222, 322, 522), sich in einem solchen Abstand befindet, der kleiner ist, als der halbe Außendurchmesser des Austragsrohrs (240) unmittelbar oberhalb der Verbindungsstelle des drehbaren Zylinders (20, 120, 220, 320, 420, 520) mit dem Austragsrohr (240). Dadurch wird die Montage des drehbaren Zylinders (20, 120, 220, 320, 420, 520) erleichtert. Wenn der halbe Außendurchmesser des Austragsrohr (240) geringfügig größer ist als der Abstand der Mitte der Verbindungsstelle des drehbaren Zylinders (20, 120, 220, 320, 420, 520) mit dem Austragsrohr (240), dann presst das Austragsrohr (240) den drehbaren Zylinder (20, 120, 220, 320, 420, 520) in die Lager (22, 122, 222, 322, 522). Dadurch wird einerseits das Herausrutschen des drehbaren Zylinders verhindert und andererseits wird der Zylinder (20, 120, 220, 320, 420, 520) massiv in die Halterung (22, 122, 222, 322, 522) gepresst.

Es kann erfindungsgemäß vorgesehen sein, dass die Öffnungen des drehbaren Zylinders (20, 120, 220, 320, 420, 520) mit den Öffnungen (12, 112, 212, 312, 512) der Kartuschenköpfe (5, 105, 205, 305, 505) mindestens eine für fließfähige Materialien durchlässige Verbindung zwischen den Innenräumen der Kartuschen (1,101, 201, 301, 501) bildet, wenn sich die Achse des Austragsrohrs (240) parallel zu den Längsachsen der Kartuschen (1, 101, 201, 301, 501) befindet und sich die Austragsrohrspitze (242) des Austragsrohrs (240) mit seinem Auslass in der den Kartuschenköpfen (5, 105, 205, 305, 505) entgegen gesetzten Richtung befindet

Erfindungsgemäß kann es auch vorgesehen sein, dass die Öffnungen des drehbaren Zylinders (20, 120, 220, 320, 420, 520) mit den Öffnungen (12, 112, 212, 312, 512) der Kartuschenköpfe (5, 105, 205, 305, 505) mindestens eine für pastöse Materialien nicht durchlässige Verbindung zwischen den Innenräumen der Kartuschen (1, 101, 201, 301, 501) bilden, wenn sich das Austragsrohr (240) im Bereich zwischen den Kartuschen (1, 101, 201, 301, 501) befindet und sich die Austragsrohrspitze (242) des Austragsrohrs (240) in der den Kartuschenböden (9, 109, 209, 309, 509) zugewandten Richtung befindet.

Bei einem Kartuschensystem für mehrere Komponenten mit dem erfindungsgemäßen Kartuschenverschlusssystem kann vorgesehen sein, dass
a) um einen inneren Hohlzylinder oder einen inneren unregelmäßig oder regelmäßig geformten Hohlkörper zwei oder mehrere Kartuschen (101, 201, 301, 501) angeordnet sind, deren Längsachsen parallel zur Achse des inneren Hohlzylinders oder des unregelmäßig oder regelmäßig geformten Hohlkörpers sind,
b) in den Kartuschenköpfen (105, 205, 305, 505) eine oder mehrere Öffnungen (112, 212, 312, 512) angeordnet sind,
c) ein Austragsrohr (240) angeordnet ist,
d) das Austragsrohr (240) mit einem Ende mit einem drehbaren Zylinder (120, 220, 320, 420, 520) verbunden ist,
e) der drehbare Zylinder (120, 220, 320, 420, 520) mindestens zwei miteinander verbundene Öffnungen besitzt, wobei mindestens eine Öffnung durchgängig mit dem Austragsrohr (240) verbunden ist,
f) der drehbare Zylinder (120, 220, 320, 420, 520) in mindestens zwei jochförmigen Lagern (122, 222, 322, 522) gelagert ist, die mit zumindest zwei Kartuschenköpfen (105, 205, 305, 505) verbunden sind,
g) mindestens eine Öffnung (112, 212, 312, 512) in den Kartuschenköpfen (105, 205, 305, 505) vorhanden ist, die mit dem Innenraum mindestens einer der Kartuschen (101, 201, 301, 501) durchlässig verbunden ist,
h) das mit dem drehbaren Zylinder (120, 220, 320, 420, 520) verbundene Austragsrohr (240) zwischen den jochförmigen Lagern (122, 222, 322, 522) angeordnet ist,
i) der Zylinder (120, 220, 320, 420, 520) um seine Zylinderachse (A) um 180 ° drehbar angeordnet ist,
j) die Kartuschen (101, 201, 301, 501) mit Förderkolben (110, 210, 310, 510) verschlossen sind,
k) die Förderkolben (110, 210, 310, 510) an der dem Kartuschenboden (109, 209, 309, 509) abgewandten Seite durch einen Steg (111, 211, 311) oder mehrere Stege (111, 211, 311) miteinander verbunden sind,
l) im inneren Hohlzylinder oder inneren unregelmäßig oder regelmäßig geformten Hohlkörper eine Stange (350) in Längsrichtung der Förderkolben (110, 210, 310, 510) angeordnet ist, die an zumindest einem Steg (111, 211, 311) mit einem Ende befestigt ist, und eine Länge von mindestens der Länge der Förderkolben (110, 210, 310, 510) hat,
m) die Stange (350) an der dem Kartuschenkopf (105, 205, 305, 505) zugewandten Seite gezahnt ist,
n) die Stange (350) einen Querschnitt kleiner dem Querschnitt des inneren Hohlzylinders oder des inneren unregelmäßig oder regelmäßig geformten Hohlkörpers hat,
o) der innere Hohlzylinder und die Kartuschen (101, 201, 301, 501) bis zur Hälfte ihrer Länge durch mindestens einen Schlitz (358) verbunden sind, der einen Querschnitt kleiner als die Querschnitte des Stegs (111, 211, 311) oder der Stege (111, 211, 311) hat, und
p) am Ende des inneren Hohlzylinders oder des inneren unregelmäßig oder regelmäßig geformten Hohlkörpers an der dem Kartuschenboden (109, 209, 309, 509) zugewandten Seite eine flexible Rastung (358) angeordnet ist, die einen Querschnitt kleiner oder gleich der gezahnten Stange (350) hat.

### Bezugszeichenliste

- 1, 101, 201, 301, 501: Kartusche
- 3, 103, 203, 303, 503: Kartuschenwand
- 5, 105, 205, 305, 506: Kartuschenkopf
- 9, 109, 209, 309, 509: Kartuschenboden
- 10,110, 210, 310, 510: Förderkolben
- 111, 211, 311: Steg
- 12, 112, 212, 312, 512: Öffnung
- 20, 120, 220, 320, 420, 520: Ventil / Zylinder
- 22, 122, 222, 322, 522: Halterung / Lager
- 24, 124, 224, 324, 424: Stutzen
- 20, 120, 220, 320, 420: Auslassöffnung
- 28, 128, 228, 328, 428: Befestigungsmittel / Gewinde
- 30, 130, 230, 330, 430, 530: Durchgang
- 240: Austragsrohr
- 242: Austragsrohrspitze
- 244: Befestigungsmittel
- 246: Mischer
- 350: Stange
- 352: Rastmittel
- 354: Gegenrastmittel
- 356: Steg
- 358: Schlitz
- A: Drehachse

## Patentansprüche

1. Kartuschensystem zum Applizieren eines Materials, insbesondere eines medizinischen Zements, umfassend zumindest zwei parallel zueinander angeordnete Kartuschen (1, 101, 201, 301, 501), wobei die zumindest zwei Kartuschen (1, 101, 201, 301, 501) Kartuschenwände (3, 103, 203, 303, 503) und jeweils einen Kartuschenkopf (5, 105, 205, 305, 505) mit jeweils zumindest einer Öffnung (12, 112, 212, 312, 512) im Kartuschenkopf (5, 105, 205, 305, 505) umfassen und an den Kartuschenköpfen (5, 105, 205, 305, 505) eine Halterung (22, 122, 222, 322, 522) angeordnet ist, **dadurch gekennzeichnet, dass**
in der Halterung (22, 122, 222, 322, 522) ein Ventil (20, 120, 220, 320, 420, 520) drehbar gelagert ist, wobei das Ventil (20, 120, 220, 320, 420, 520) mit einer Auslassöffnung (26, 126, 226, 326, 426) verbundene Durchgänge (30, 130, 230, 330, 430, 530) durch das Ventil (20, 120, 220, 320, 420, 520) umfasst, das Ventil (20, 120, 220, 320, 420, 520) in einer geschlossenen Position alle Öffnungen (12, 112, 212, 312, 512) jeder Kartusche (1, 101, 201, 301, 501), dicht verschließt, in einer offenen Position des Ventils (20, 120, 220, 320, 420, 520) die Durchgänge (30, 130, 230, 330, 430, 530) mit den Öffnungen (12, 112, 212, 312, 512) verbunden sind, so dass der Kartuscheninhalt aus den Kartuschen (1, 101, 201, 301, 501) durch die Auslassöffnung (26, 126, 226, 326, 426) drückbar ist, und wobei das Ventil (20, 120, 220, 320, 420, 520) von der geschlossenen Position in die offene Position durch eine Drehung des Ventils (20, 120, 220, 320, 420, 520) überführbar ist,
am Ventil (20, 120, 220, 320, 420, 520) ein Austragsrohr (240) angeordnet ist, das an der Auslassöffnung (26, 126, 226, 326, 426) beginnt und die Durchgänge (30, 130, 230, 330, 430, 530) bis zu einer Austragsrohrspitze (242) verlängert, und
das Austragsrohr (240) bei geschlossenem Ventil (20, 120, 220, 320, 420, 520) parallel zu und zwischen den zumindest zwei Kartuschen (1, 101, 201, 301, 501) angeordnet ist.

2. Kartuschensystem nach Anspruch 1, **dadurch gekennzeichnet, dass**
jede Kartusche (1, 101, 201, 301, 501) zumindest einen Förderkolben (10, 110, 210, 310, 510) gegenüber des Kartuschenkopfs (5, 105, 205, 305, 505) zum Austreiben des Kartuscheninhalts durch die Öffnung (12, 112, 212, 312, 512) umfasst, der die Kartuschen (1, 101, 201, 301, 501) bodenseitig, insbesondere gasdicht, verschließt.

3. Kartuschensystem nach Anspruch 1 oder 2, **dadurch gekennzeichnet, dass**
an mehreren Öffnungen im Ventil (20, 120, 220, 320, 420, 520) Dichtungsringe angeordnet sind, so dass bei geöffnetem Ventil (20, 120, 220, 320, 420, 520) fluiddichte Verbindungen von den Öffnungen (12, 112, 212, 312, 512) in den Kartuschen (1, 101, 201, 301, 501) zu den Durchgängen (30, 130, 230, 330, 430, 530) bereitgestellt ist oder sind.

4. Kartuschensystem nach einem der Ansprüche 1 bis 3, **dadurch gekennzeichnet, dass** am Ventil (20, 120, 220, 320, 420, 520) ein Stutzen (24, 124, 224, 324, 424) angeordnet ist, wobei der Stutzen (24, 124, 224, 324, 424) ein Befestigungsmittel (28, 128, 228, 328, 428) zum Befestigen eines Austragsrohrs (240) umfasst, der Durchgang (30, 130, 230, 330, 430, 530) sich durch den Stutzen (24, 124, 224, 324, 424) erstreckt und die Auslassöffnung (26, 126, 226, 326, 426) am Stutzen (24, 124, 224, 324, 424) angeordnet ist, und am Stutzen ein Austragsrohr (240) angeordnet ist.

5. Kartuschensystem nach Anspruch 4, **dadurch gekennzeichnet, dass**
das Befestigungsmittel (28, 128, 228, 328, 428) ein Gewinde, insbesondere ein Außengewinde (28, 128, 228, 428) ist.

6. Kartuschensystem nach einem der Ansprüche 4 oder 5, **dadurch gekennzeichnet, dass**
das Austragsrohr (240) ein Befestigungsmittel (244), insbesondere ein Innengewinde (244), umfasst, mit dem das Austragsrohr (240) am Befestigungsmittel (244) des Stutzens (24, 124, 224, 324, 424) lösbar verbunden ist oder das Austragsrohr (240) fest mit dem Ventil (20, 120, 220, 320, 420, 520) verbunden ist.

7. Kartsuchensystem nach einem der vorangehenden Ansprüche, **dadurch gekennzeichnet, dass**
parallel zu den zumindest zwei Kartuschen (1, 101, 201, 301, 501), vorzugsweise zwischen zumindest zwei Kartuschen (1, 101, 201, 301, 501), besonders bevorzugt in einem Hohlkörper, um den mehrere Kartuschen (1, 101, 201, 301, 501) parallel angeordnet sind, eine bewegliche Stange (350) parallel zu den Förderkolben (10, 110, 210, 310, 510) angeordnet ist, die mit den Förderkolben (10, 110, 210, 310, 510) über zumindest einen Steg (356) und/oder eine Platte fest verbunden ist, wobei die Stange (350) an der dem Ventil (20, 120, 220, 320, 420, 520) zugewandten Seite ein Rastmittel (352) umfasst und an der Kartuschenwand (3, 103, 203, 303, 503) oder den Kartuschenwänden (3, 103, 203, 303, 503), oder bevorzugt an der Innenwand des Hohlkörpers, ein Gegenrastmittel (354) angebracht ist, das mit dem Rastmittel (352) der Stange (350) derart zusammenwirkt, dass eine Bewegung der Stange (350) in Richtung des Kartuschenbodens (9, 109, 209, 309, 509) und damit der Förderkolben (10, 110, 210, 310, 510) aus den Kartuschen (1, 101, 201, 301, 501) heraus deutlich erschwert, insbesondere verhindert ist.

8. Kartuschensystem nach Anspruch 7, **dadurch gekennzeichnet, dass**
die Kartuschenwände (3, 103, 203, 303, 503) zumindest einen Schlitz (358) beginnend am Kartuschenboden (9, 109, 209, 309, 509) aufweisen, der parallel zur Stange (350) angeordnet ist, wobei die Breite des oder der Schlitze (358) zur Aufnahme des oder der Stege (356) ausreicht und insbesondere die Länge des oder der Schlitze (358) bis mindestens zur Hälfte der Kartuschenlänge reicht.

9. Kartuschensystem nach einem der vorangehenden Ansprüche, **dadurch gekennzeichnet, dass**
das Austragsrohr (240) einen statischen Mischer (246) umfasst.

10. Kartuschensystem nach einem der vorangehenden Ansprüche, **dadurch gekennzeichnet, dass**
das Ventil (20, 120, 220, 320, 420, 520) in der offenen und der geschlossenen Position in Presspassung über den Öffnungen (12, 112, 212, 312, 512) an den Kartuschenköpfen (5, 105, 205, 305, 505) angeordnet ist und diese dicht abschließt oder dicht mit den Durchgängen (30, 130, 230, 330, 430, 530) verbindet.

11. Kartuschensystem nach einem der vorangehenden Ansprüche, **dadurch gekennzeichnet, dass**
das Kartuschensystem zumindest drei parallel zueinander angeordnete Kartuschen (1, 101, 201, 301, 501) zum Mischen und Applizieren eines Mischguts, insbesondere eines medizinischen Zements, umfasst.

12. Kartuschensystem nach Anspruch 11, **dadurch gekennzeichnet, dass**
die Durchgänge (30, 130, 230, 330, 430, 530) im Ventil (20, 120, 220, 320, 420, 520) die Öffnungen (12, 112, 212, 312, 512) der zumindest zwei Kartuschen (1, 101, 201, 301, 501) mit der Auslassöffnung (26, 126, 226, 326, 426) verbinden, wenn das Ventil (20, 120, 220, 320, 420, 520) in der geöffneten Position ist.

13. Kartuschensystem nach einem der vorangehenden Ansprüche, **dadurch gekennzeichnet, dass**
das Ventil (20, 120, 220, 320, 420, 520) zylindrisch geformt ist, die Form eines Zylinders mit elliptischer Grundfläche aufweist oder die Form eines Zylinderabschnitts hat.

## Claims

1. Cartridge system for application of a material, in particular a medical cement, comprising at least two cartridges (1, 101, 201) arranged parallel to each other, whereby the at least two cartridges (1, 101, 201, 301, 501) comprise cartridge walls (3, 103, 203, 303, 503) and one cartridge head (5, 105, 205, 305, 505) each having at least one opening (12, 112, 212, 312, 512) in each cartridge head (5, 105, 205, 305, 505), and a bracket (22, 122, 222, 322, 522) is arranged on the cartridge heads (5, 105, 205, 305, 505), **characterised in that**
a valve (20, 120, 220, 320, 420, 520) being mounted in rotatable manner in the bracket (22, 122, 222, 322, 522), whereby the valve (20, 120, 220, 320, 420, 520) comprises passages (30, 130, 230, 330, 430, 530) through the valve (20, 120, 220, 320, 420, 520) that is connected to an outlet opening (26, 126, 226, 326, 426), the valve (20, 120, 220, 320, 420, 520), in a closed position, closing all openings (12, 112, 212, 312, 512) of each cartridge (1, 101, 201, 301, 501), in a sealed manner, the passages (30, 130, 230, 330, 430, 530) being connected to the openings (12, 112, 212, 312, 512) in an open position of the valve (20, 120, 220, 320, 420, 520) such that the cartridge content can be squeezed out of the cartridges (1, 101, 201, 301, 501) through the outlet opening (26, 126, 226, 326, 426), and whereby the valve (20, 120, 220, 320, 420, 520) can be transitioned from the closed position to the open position through a rotation of the valve (20, 120, 220, 320, 420, 520), the valve (20, 120, 220, 320, 420, 520) has a dispensing tube (240) arranged on it that starts on the outlet opening (26, 126, 226, 326, 426) and extends the passages (30, 130, 230, 330, 430, 530) up to a dispensing tube tip (242), and the dispensing tube (240), with the valve (20, 120, 220, 320, 420, 520) being closed, is arranged parallel to and in between the at least two cartridges (1, 101, 201, 301, 501).

2. Cartridge System according to Claim 1, **characterised in that**
each cartridge (1, 101, 201, 301, 501) comprises at least one feed plunger (10, 110, 210, 310, 510) opposite from the cartridge head (5, 105, 205, 305, 505) for expelling the cartridge content through the opening (12, 112, 212, 312, 512), which feed plunger closes the cartridges (1, 101, 201, 301, 501) on the floor side, in particular in a gastight manner.

3. Cartridge System according to Claim 1 or 2, **characterised in that**
sealing rings are arranged on openings in the valve (20, 120, 220, 320, 420, 520) such that, with the valve (20, 120, 220, 320, 420, 520) being open, fluid-tight connections from the openings (12, 112, 212, 312, 512) in the cartridges (1, 101, 201, 301, 501) to passages (30, 130, 230, 330, 430, 530) are provided.

4. Cartridge System according to any one of the Claims 1 to 3, **characterised in that**
a nozzle (24, 124, 224, 324, 424) is arranged on the valve (20, 120, 220, 320, 420, 520), whereby the nozzle (24, 124, 224, 324, 424) comprises a fastening means (28, 128, 228, 328, 428) for fastening a dispensing tube (240), the passage (30, 130, 230, 330, 430, 530) extending through the nozzle (24, 124, 224, 324, 424), and the outlet opening (26, 126, 226, 326, 426) is arranged on the nozzle (24, 124, 224, 324, 424), and a dispensing tube (240) is arranged on the nozzle (24, 124, 224, 324, 424).

5. Cartridge System according to Claim 4, **characterised in that**
the fastening means (28, 128, 228, 328, 428) is a thread, in particular an external thread (28, 128, 228, 428).

6. Cartridge System according to Claim 4 or 5, **characterised in that**
the dispensing tube (240) comprises a fastening means (244), in particular an internal thread, by which the dispensing tube (240) is detachably connected at the fastening means (28, 128, 228, 328, 428) of the nozzle (24, 124, 224, 324, 424) or the dispensing tube (240) being fixedly connected to the valve (20, 120, 220, 320, 420, 520).

7. Cartridge System according to any one of the preceding Claims, **characterised in that** a mobile rod (350) is arranged parallel to the at least two cartridges (1, 101, 201, 301, 501), preferably in between at least two cartridges (1, 101, 201, 301, 501), particularly preferably in a hollow body about which multiple cartridges (1, 101, 201, 301, 501) are arranged to be parallel, the mobile rod (350) being arranged parallel to the feed plungers (10, 110, 210, 310, 510) and fixedly connected to the feed plungers (10, 110, 210, 310, 510) through at least one fin (356) and/or one plate, whereby the rod (350) comprises a snap-in locking means (352) on the side facing the valve (20, 120, 220, 320, 420, 520) and an opposite snap-in locking means (354) is attached at the cartridge wall (3, 103, 203, 303, 503) or cartridge walls (3, 103, 203, 303, 503) or preferably on the internal wall of the hollow body, and acts in concert with the snap-in locking means (352) of the rod (350) in a manner such that a motion of the rod (350) in the direction of the cartridge floor (9, 109, 209, 309, 509) and therefore a motion of the feed plungers (10, 110, 210, 310, 510) out of the cartridges (1, 101, 201, 301, 501) is significantly hindered, in particular is prevented.

8. Cartridge System according to Claim 7, **characterised in that**
the cartridge walls (3, 103, 203, 303, 503) comprise at least one slit (358) that commences at the cartridge floor (9, 109, 209, 309, 509) and is arranged parallel to the rod (350), whereby the width of the slit or slits (358) is sufficient to take up the fin or fins (356) and the length of the slit or slits (358) extends, in particular, to at least half of the length of the cartridges (1, 101, 201, 301, 501).

9. Cartridge System according to any one of the preceding Claims, **characterised in that** the dispensing tube (240) comprises a static mixer (246).

10. Cartridge System according to any one of the preceding Claims, **characterised in that** the valve (20, 120, 220, 320, 420, 520), in the open and in the closed position, is arranged in a press-fit manner over the openings (12, 112, 212, 312, 512) on the cartridge heads (5, 105, 205, 305, 505) and closes these in a sealed manner or connects them in a sealed manner to the passages (30, 130, 230, 330, 430, 530).

11. Cartridge System according to any one of the preceding Claims, **characterised in that** the cartridge system comprises at least three cartridges (1, 101, 201, 301, 501), which are arranged to be parallel to each other, for mixing and applying a mixing ware, in particular a medical cement.

12. Cartridge System according to Claim 11, **characterised in that**
the passages (30, 130, 230, 330, 430, 530) in the valve (20, 120, 220, 320, 420, 520) connect the openings (12, 112, 212, 312, 512) of at least two cartridges (1, 101, 201, 301, 501) to the outlet opening (26, 126, 226, 326, 426) when the valve (20, 120, 220, 320, 420, 520) is in the open position.

13. Cartridge System according to any one of the preceding Claims, **characterised in that** the valve (20, 120, 220, 320, 420, 520) is cylinder-shaped, takes the shape of a cylinder with an elliptical base or the shape of a section of a cylinder.

## Revendications

1. Système de cartouches permettant l'application d'un matériau, notamment d'un ciment médical, comprenant au moins deux cartouches (1, 101, 201, 301, 501) disposées parallèles l'une à l'autre, où les au moins deux cartouches (1, 101, 201, 301, 501) comprennent des parois de cartouche (3, 103, 203, 303, 503) et respectivement une tête de cartouche (5, 105, 205, 305, 505) avec respectivement au moins un orifice (12, 112, 212, 312, 512) dans la tête de cartouche (5, 105, 205, 305, 505), et un support (22, 122, 222, 322, 522) est disposé sur les têtes de cartouche (5, 105, 205, 305, 505), **caractérisé en ce**
**qu'**une soupape (20, 120, 220, 320, 420, 520) est logée en pouvant être mise en rotation dans le support (22, 122, 222, 322, 522), où la soupape (20, 120, 220, 320, 420, 520) comprend des passages (30, 130, 230, 330, 430, 530) reliés avec un orifice de sortie (26, 126, 226, 326, 426) par la soupape (20, 120, 220, 320, 420, 520), la soupape (20, 120, 220, 320, 420, 520) ferme de manière étanche tous les orifices (12, 112, 212, 312, 512) de chaque cartouche (1, 101, 201, 301, 501) dans une position fermée, dans une position ouverte de la soupape (20, 120, 220, 320, 420, 520), les passages (30, 130, 230, 330, 430, 530) sont reliés avec les orifices (12, 112, 212, 312, 512), de sorte que le contenu de cartouche peut être pressé hors des cartouches (1, 101, 201, 301, 501) par l'orifice de sortie (26, 126, 226, 326, 426), et où la soupape (20, 120, 220, 320, 420, 520) peut être transférée de la position fermée dans la position ouverte par une rotation de la soupape (20, 120, 220, 320, 420, 520),
un tube de sortie (240) est disposé sur la soupape (20, 120, 220, 320, 420, 520), tube qui commence au niveau de l'orifice de sortie (26, 126, 226, 326, 426) et prolonge les passages (30, 130, 230, 330, 430, 530) jusqu'à une pointe de tube de sortie (242), et le tube de sortie (240), pour une soupape (20, 120, 220, 320, 420, 520) fermée, est disposé parallèlement et entre les au moins deux cartouches (1, 101, 201, 301, 501).

2. Système de cartouches selon la revendication 1, **caractérisé en ce que**
chaque cartouche (1, 101, 201, 301, 501) comprend au moins un piston de transport (10, 110, 210, 310, 510) en face de la tête de cartouche (5, 105, 205, 305, 505) permettant d'expulser le contenu de cartouche par l'orifice (12, 112, 212, 312, 512), qui ferme, notamment de manière étanche aux gaz, les cartouches (1, 101, 201, 301, 501) du côté du fond.

3. Système de cartouches selon la revendication 1 ou la revendication 2, **caractérisé en ce que**
des bagues d'étanchéité sont disposées au niveau de plusieurs orifices dans la soupape (20, 120, 220, 320, 420, 520), de sorte que, pour une soupape (20, 120, 220, 320, 420, 520) ouverte, des liaisons étanches vis-à-vis des fluides sont établies ou sont réalisées à partir des orifices (12, 112, 212, 312, 512) dans les cartouches (1, 101, 201, 301, 501) vers les passages (30, 130, 230, 330, 430, 530).

4. Système de cartouches selon l'une des revendications 1 à 3, **caractérisé en ce**
**qu'**un embout (24, 124, 224, 324, 424) est disposé sur la soupape (20, 120, 220, 320, 420, 520), où l'embout (24, 124, 224, 324, 424) comprend un système de fixation (28, 128, 228, 328, 428) pour la fixation d'un tube de sortie (240), le passage (30, 130, 230, 330, 430, 530) s'étend à travers l'embout (24, 124, 224, 324, 424), et l'orifice de sortie (26, 126, 226, 326, 426) est disposé sur l'embout (24, 124, 224, 324, 424), et un tube de sortie (240) est disposé sur l'embout.

5. Système de cartouches selon la revendication 4, **caractérisé en ce que**
le système de fixation (28, 128, 228, 328, 428) est un filetage, notamment un filetage extérieur (28, 128, 228, 428).

6. Système de cartouches selon l'une des revendications 4 ou 5, **caractérisé en ce que**
le tube de sortie (240) comprend un système de fixation (244), notamment un filetage intérieur (244), avec lequel le tube de sortie (240) est relié de manière amovible sur le système de fixation (244) de l'embout (24, 124, 224, 324, 424), ou le tube de sortie (240) est relié solidement avec la soupape (20, 120, 220, 320, 420, 520).

7. Système de cartouches selon l'une des revendications précédentes, **caractérisé en ce que**,
parallèlement par rapport aux au moins deux cartouches (1, 101, 201, 301, 501), de préférence entre les au moins deux cartouches (1, 101, 201, 301, 501), de manière particulièrement préférée dans un corps creux, autour duquel plusieurs cartouches (1, 101, 201, 301, 501) sont disposées parallèlement, une tige (350) mobile, parallèle aux pistons de transport (10, 110, 210, 310, 510) est disposée, qui est reliée solidement avec le piston de transport (10, 110, 210, 310, 510) par le biais d'au moins un pontage (356) et/ou une plaque, où la tige (350) comprend un organe de butée (352) sur le côté orienté vers la soupape (20, 120, 220, 320, 420, 520) et un organe de butée complémentaire (354) est rapporté sur la paroi de cartouche (3, 103, 203, 303, 503) ou sur les parois de cartouches (3, 103, 203, 303, 503), ou de préférence, sur la paroi intérieure du corps creux, qui agit conjointement avec l'organe de butée (352) de la tige (350) de sorte qu'un déplacement de la tige (350) en direction du fond de cartouche (9, 109, 209, 309, 509) est notamment empêché, et ainsi le piston de transport (10, 110, 210, 310, 510) hors des cartouches (1, 101, 201, 301, 501) est rendu nettement plus difficile.

8. Système de cartouches selon la revendication 7, **caractérisé en ce que**
les parois de cartouche (3, 103, 203, 303, 503) présentent au moins une fente (358) commençant au niveau du fond de cartouche (9, 109, 209, 309, 509), qui est disposée parallèlement par rapport à la tige (350), où la largeur de la fente ou des fentes (358) est suffisante pour l'admission du ou des pontages (356) et notamment la longueur de la fente ou des fentes (358) va jusqu'au moins la moitié de la longueur de la cartouche.

9. Système de cartouches selon l'une des revendications précédentes, **caractérisé en ce que**
le tube de sortie (240) comprend un mélangeur (246) statique.

10. Système de cartouches selon l'une des revendications précédentes, **caractérisé en ce que**
la soupape (20, 120, 220, 320, 420, 520), dans la position ouverte et dans la position fermée, est disposée dans un ajustement serré sur les têtes de cartouche (5, 105, 205, 305, 505) par le biais des orifices (12, 112, 212, 312, 512) et ferme ces deniers de manière étanche ou les relie de manière étanche avec les passages (30, 130, 230, 330, 430, 530).

11. Système de cartouches selon l'une des revendications précédentes, **caractérisé en ce que**
le système de cartouches comprend au moins trois cartouches (1, 101, 201, 301, 501) disposées parallèles les unes aux autres pour le mélange et l'application d'un produit mélangé, notamment un ciment médical.

12. Système de cartouches selon la revendication 11, **caractérisé en ce que**
les passages (30, 130, 230, 330, 430, 530) dans la soupape (20, 120, 220, 320, 420, 520) relient les orifices (12, 112, 212, 312, 512) des au moins deux cartouches (1, 101, 201, 301, 501) avec l'orifice de sortie (26, 126, 226, 326, 426) lorsque la soupape (20, 120, 220, 320, 420, 520) est dans la position ouverte.

13. Système de cartouches selon l'une des revendications précédentes, **caractérisé en ce que**
la soupape (20, 120, 220, 320, 420, 520) est de forme cylindrique, présente la forme d'un cylindre avec une base elliptique ou a la forme d'un tronçon de cylindre.
